(19) Europäisches Patentamt
European Patent Office
Office européen des brevets

(11) Veröffentlichungsnummer: **0 001 423**
**A1**

# (12) EUROPÄISCHE PATENTANMELDUNG

(21) Anmeldenummer: **78100978.2**

(22) Anmeldetag: **25.09.78**

(51) Int. Cl.²: **A 01 N 9/22,** C 07 D 487/04 // (C07D487/04, 241/00, 239/00)

(30) Priorität: **06.10.77 DE 2745007**

(43) Veröffentlichungstag der Anmeldung: **18.04.79 Patentblatt 79/8**

(84) Benannte Vertragsstaaten: **BE CH DE FR GB NL**

(71) Anmelder: **Bayer Aktiengesellschaft, Zentralbereich Patente,Marken und Lizenzen Bayerwerk, D-5090 Leverkusen 1 (DE)**

(72) Erfinder: **Seng, Florin, Dr., Am Katterbach 46, D-5060 Bergisch-Gladbach 2 (DE)**
Erfinder: **Scheinpflug, Hans, Dr., Am Thelenhof 15, D-5090 Leverkussen 1 (DE)**
Erfinder: **Kraus, Peter, Dr., Düsseldorfer Strasse 43, D-5000 Köln 80 (DE)**

## (54) Mittel zur Bekämpfung von Pflanzenbakteriosen.

(57) Die Erfindung betrifft die Verwendung von speziellen bekannten 4-Alkylamino-pyrimido-(4,5-b)-chinoxylin-5,10-dioxiden zur Bekämpfung von pflanzenschädigenden Bakterien.

Die Verbindungen der allgemeinen Formel

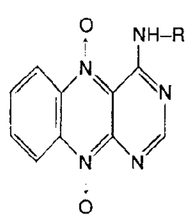

in welcher

R für Methyl oder Äthyl steht.

weisen starke bakterizide Wirkungen auf. Sie können als Pflanzenschutzmittel Verwendung finden zur Behandlung oberirdischer Pflanzenteile. zur Saatgutbehandlung und zur Bodenbehandlung.

EP 0 001 423 A1

BAYER AKTIENGESELLSCHAFT
Zentralbereich
Patente, Marken und Lizenzen

5090 Leverkusen, Bayerwerk
Slr/AB
IIb

Mittel zur Bekämpfung von Pflanzenbakteriosen

Die vorliegende Erfindung betrifft die Verwendung von speziellen bekannten 4-Alkylamino-pyrimido-(4,5-b)-chinoxalin-5,10-dioxiden zur Bekämpfung von pflanzenschädigenden Bakterien.

Durch pathogene Bakterien verursachte Pflanzenkrankheiten breiten sich in zunehmendem Maße aus, so daß bereits jetzt die Anbauwürdigkeit einiger Kulturpflanzen in bestimmten Regionen wirtschaftlich gefährdet ist. Bedeutende Erreger entstammen der Familie der Pseudomonadaceae, z.B. Pseudomonas solanacearum, Pseudomonas lachrymans, Pseudomonas syringae, Xanthomonas citri, Xanthomonas oryzae, Xanthomonas vesicatoria,der Familie der Enterobacteriaceae, z.B. Erwinia amylovora, bzw. der Familie der Corynebacteriaceae. Die bisher zur Verfügung stehenden Möglichkeiten zur Bekämpfung bzw. Verhütung dieser Krankheiten sind sehr aufwendig und dabei noch unzureichend. Im praktischen Einsatz befinden sich hierfür zumeist Antibiotika, deren Herstellung teuer ist und die vielfachtoxisch und unter atmosphärischen Bedingungen nur kurze Zeit stabil sind. Ferner dienen noch

- wie schon lange bekannt - basische Kupfersalze, wie z.B. Kupferoxychlorid, oder Kupferhydroxid-Kupferchlorid-Mischungen -, zur Bekämpfung von Pflanzenkrankheiten (R. Wegler, Chemie der Pflanzenschutz- und Schädlingsbekämpfungsmittel, Band 2, Springer-Verlag, Heidelberg (1970)).

Wie weiterhin bekannt ist, besitzt die Gruppe der 4-Alkylamino-pyrimido-(4,5-b)-chinoxalin-5,10-dioxide eine Wirksamkeit gegen gramnegative und grampositive Bakterien (vgl. hierzu DT-OS 2 034 476 [Le A 12 963]), Verbindungen aus dieser Gruppe können sowohl in der Humanals auch in der Tiermedizin zur Prophylaxe und zur Behandlung von durch Bakterien hervorgerufenen Infektionen Verwendung finden. Über einen Einsatz dieser Verbindungen als Pflanzenschutzmittel wurde bisher noch nichts bekannt.

Es wurde nun gefunden, daß speziell diejenigen Verbindungen aus der Gruppe der 4-Alkylamino-pyrimido-(4,5-b)-chinoxalin-5,10-dioxide der allgemeinen Formel

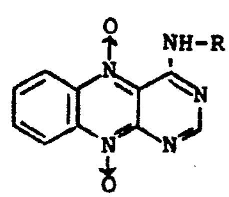

(I)

in welcher

R für Methyl oder Äthyl steht,

eine besonders gute Wirksamkeit gegen pflanzenschädigende Bakterien besitzen.

Es ist als überraschend anzusehen, daß speziell die Methyl- und die Äthyl-Verbindungen aus der Reihe der 4-Alkylamino-pyrimido-(4,5-b)-chinoxalin-5,10-dioxide der Formel I eine für den Pflanzenschutz interessante bakterizide Wirksamkeit besitzen. Die beiden erfindungsgemäß verwendeten Verbindungen übertreffen auch das als Standardpräparat weit verbreitete basische Kupferchlorid. Sie stellen daher eine Bereicherung der Technik dar.

Die erfindungsgemäß zu verwendenden beiden Verbindungen sind durch die obige Formel I genau definiert. Die Verbindungen sind bekannt, ebenso deren Herstellung (vgl. DT-OS 2 034 776 /Le A 12 963/) aus dem 2-Cyano-3-(dimethylamino-formylimino)-chinoxalin-di-N-oxid der Formel

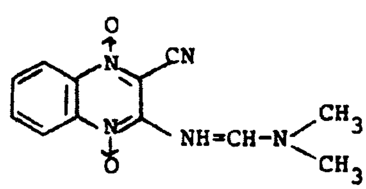

(II)

und Methyl- oder Äthylamin (siehe auch die Herstellungsbeispiele).

Die erfindungsgemäß verwendbaren Wirkstoffe weisen starke bakterizide Wirkungen auf. Sie können als Pflanzenschutzmittel Verwendung finden, zur Behandlung oberirdischer Pflanzenteile, zur Saatgutbehandlung und zur Bodenbehandlung.

Bakterizide Mittel werden im Pflanzenschutz eingesetzt zur Bekämpfung von Bakterien aus der Familie der Pseudomonadaceae, z.B. von Pseudomonas solanacearum, Pseudomonas lachrymans, Pseudomonas syringae, Xanthomonas citri, Xanthomonas oryzae und Xanthomonas vesicatoria, der Familie der Enterobacteriaceae, z.B. von Erwinia amylovora, bzw. der Familie der Corynebacteriaceae, ferner aus der Familie der Rhizobiaceae, z.B. von Agrobacterium tumefaciens.

Die erfindungsgemäß verwendbaren Stoffe sind gut pflanzenverträglich.

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsionskonzentrate, Saatgutpuder, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä. sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B.

auch organische Lösungsmittel als Hilfslösungsmittel verwendet werden. Als flüssige Lösungsmittel kommen im wesentlichen infrage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe: natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate: gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehle, Kokosnußschalen, Maiskolben und Tabakstengel; als Emulgier- und/oder schaumerzeugende Mittel: nichtionogene und anionische Emulgatoren, wie Polyoxyäthylen-Fettsäure-Ester, Polyoxyäthylen-Fettalkohol-Äther, z.B. Alkylaryl-polyglykol-äther, Alkylsulfonate, Alkylsulfate, Arylsulfonate sowie Eiweißhydrolysate; als Dispergiermittel: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat,

Es können Farbstoffe wie anorganische Pigmente, z.B. Eisenoxid, Titanoxid, Ferrocyanblau und organische Farbstoffe, wie Alizarin-, Azo-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen, wie Fungiziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z. B. durch Gießen, Spritzen, Sprühen, Stäuben, Streuen, Trockenbeizen, Feuchtbeizen, Naßbeizen, Schlämmbeizen oder Inkrustieren.

Bei der Verwendung als Blattbakterizide können die Wirkstoffkonzentrationen in den Anwendungsformen in einem größeren Bereich variiert werden. Sie liegen im allgemeinen zwischen 0,5 und 0,0005 Gewichtsprozenten, vorzugsweise zwischen 0,2 und 0,001 %.

Bei der Saatgutbehandlung werden im allgemeinen Wirkstoffmengen von 0,01 bis 50 g je Kilogramm Saatgut, vorzugsweise 0,5 bis 5 g, benötigt.

Zur Bodenbehandlung sind Wirkstoffmengen von 1 bis 1000 g je cbm Boden, vorzugsweise von 10 bis 200 g, erforderlich.

Die Verwendungsmöglichkeiten gehen aus den nachfolgenden Beispielen hervor:

Beispiel A

Agarplatten-Test

Verwendeter Nährboden:

| | |
|---|---|
| 20 | Gew.-Teile Agar-Agar |
| 200 | Gew.-Teile Kartoffeldekokt |
| 5 | Gew.-Teile Malz |
| 15 | Gew.-Teile Dextrose |
| 5 | Gew.-Teile Pepton |
| 2 | Gew.-Teile Dinatrium-hydrogenphosphat |
| 0,3 | Gew.-Teile Calciumnitrat |

Verhältnis von Lösungsmittelgemisch zum Nährboden:

| | |
|---|---|
| 2 | Gew.-Teile Lösungsmittelgemisch |
| 100 | Gew.-Teile Agarnährboden |

Zusammensetzung Lösungsmittelgemisch:

| | |
|---|---|
| 0,19 Gew.-Teile | Dimethylformamid oder Aceton |
| 0,01 Gew.-Teile | Emulgator Alkylarylpolyglykoläther |
| 1,80 Gew.-Teile | Wasser |
| 2 Gew.-Teile | Lösungsmittelgemisch |

Man vermischt die für die gewünschte Wirkstoffkonzentration im Nährboden nötige Wirkstoffmenge mit der angegebenen Menge des Lösungsmittelgemisches. Das Konzentrat wird im genannten Mengenverhältnis mit dem flüssigen, auf 42°C abgekühlten Nährboden gründlich vermischt und in Petrischalen mit einem Durchmesser von 9 cm gegossen. Ferner werden

Kontrollplatten ohne Präparatbeimischung aufgestellt.

Ist der Nährboden erkaltet und fest, werden die Platten mit den in der Tabelle angegebenen Bakterien beimpft und bei etwa 21°C inkubiert.

Die Auswertung erfolgt je nach der Wachstumsgeschwindigkeit des Kolonienwachstums nach 4 bis 10 Tagen. Bei der Auswertung wird das Wachstum auf den behandelten Nährboden mit dem Wachstum auf dem Kontrollnährboden verglichen. Die Bonitierung des Wachstums geschieht mit folgenden Kennzahlen:

| | |
|---|---|
| 1 | kein Wachstum |
| bis 3 | sehr starke Hemmung des Wachstums |
| bis 5 | mittelstarke Hemmung des Wachstums |
| bis 7 | schwache Hemmung des Wachstums |
| 9 | Wachstum gleich der unbehandelten Kontrolle |

Wirkstoffe, Wirkstoffkonzentration und Resultate gehen aus der nachfolgenden Tabelle hervor:

**T a b e l l e   A**

Agarplatten-Test

| Wirkstoffe | Wirkstoffkonz. /ppm/ | Bakterien | | | |
|---|---|---|---|---|---|
| | | Erwinia carotovora | Xanthomonas oryzae | Xanthomonas vesicatoria | Xanthomonas malvacearum |
| Kupferoxychlorid $3\ Cu(OH)_2 \cdot CuCl_2 \cdot xH_2O$ (bekannt) | 10 | | | | 9 |
| | 25 | | 7 | | |
| | 50 | 5 | | | |
| | 100 | | | 7 | |
| 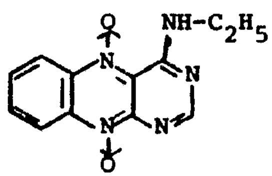 | 10 | | | | 1 |
| | 25 | | 1 | | |
| | 50 | 3 | | | |
| | 100 | | | 1 | |

Beispiel B

Bakterien-Test / Xanthomonas oryzae

| | | | |
|---|---|---|---|
| Lösungsmittel: | 11,75 | Gew.-Teile | Aceton |
| Dispergiermittel: | 0,75 | Gew.-Teile | Alkylarylpoly-glykoläther |
| Wasser: | 987,50 | Gew.-Teile | |
| andere Zusätze | - | Gew.-Teile | - |

Man vermischt die für die gewünschte Wirkstoffkonzentration in der Spritzflüssigkeit nötige Wirkstoffmenge mit der angegebenen Menge des Lösungsmittels und des Dispergiermittels und verdünnt das Konzentrat mit der angegebenen Menge Wasser.

Mit der Spritzflüssigkeit bespritzt man 30 etwa 40 Tage alte Reispflanzen bis zur Tropfnässe. Die Pflanzen verbleiben bis zum Abtrocknen in einem Gewächshaus bei Temperaturen von 22 bis 24°C und einer relativen Luftfeuchtigkeit von etwa 70 %. Danach werden Nadeln in eine wäßrige Bakteriensuspension von Xanthomonas oryzae getaucht und die Pflanzen durch Anstechen der Blätter inokuliert. Die Pflanzen stehen nach der Inokulation 24 Stunden bei 100 % relativer Luftfeuchtigkeit und danach in einem Raum bei 26 bis 28°C und 80 % relativer Luftfeuchtigkeit. 10 Tage nach der Inokulation wird der Befall bei allen durch Stich verletzten, inokulierten Blätter von vorher mit Präparat behandelten Pflanzen in Wertzahlen von 1 bis 9 ausgewertet. 1 bedeutet 100 %ige Wirkung, 3 = gute Wirkung, 5 = mäßige Wirkung und 9 = keine Wirkung.

Wirkstoffe, Wirkstoffkonzentrationen und Resultate gehen aus der nachfolgenden Tabelle hervor:

T a b e l l e   B

Bakterien-Test / Xanthomonas oryzae

| Wirkstoff | Befallswertzahl bei einer Wirkstoffkonzentration (in %) von 0,025 |
|---|---|
| Kupferoxychlorid<br>3 $Cu(OH)_2 \cdot CuCl_2 \cdot xH_2O$<br>(bekannt) | 7 |
| 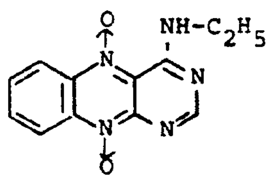 | 3 |
| 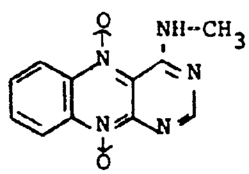 | 3 |

Herstellungsbeispiele

Beispiel 1:

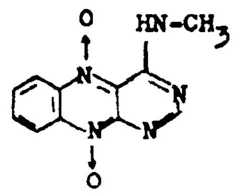

25,7 g (0,1 Mol) 2-Cyano-3-(dimethylamino-formylimino)-chinoxalin-di-N-oxid werden in 80 ml Dimethylformamid suspendiert, mit 17,7 g (0,2 Mol) 35 %iger wäßriger Methylaminlösung versetzt und auf etwa 50°C erwärmt. Nach etwa einer Stunde wird abgekühlt und abgesaugt. Man erhält 18 g (74 % der Theorie) 4-Methylamino-pyrimido-(4,5-b)-chinoxalin-5,10-dioxid in Form roter Kristalle, die nach dem Umlösen aus Dimethylformamid bei 189°C unter Zersetzung schmelzen.

Darstellung des Vorproduktes:

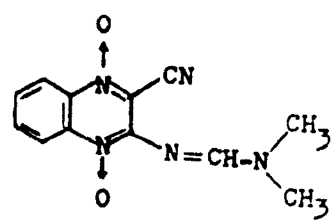

20,2 g (0,1 Mol) 2-Amino-3-cyanochinoxalin-di-N-oxid (Darstellung nach K. Ley, F. Seng, H. Eholzer, R.Nast und R. Schubart, Angew. Chemie 81, 569 (1969))werden in 100 ml Dimethylformamid suspendiert und unter Rühren und Eiskühlung tropfenweise mit 15,3 g (0,1 Mol) Phosphoroxychlorid versetzt. Nach dem Zutropfen rührt man noch eine Stunde nach

und saugt dann den Niederschlag ab. Man erhält 23 g (90 % der Theorie) 2-Cyano-3-(dimethylamino-formylimino)-chinoxalin-di-N-oxid in Form roter Kristalle, die nach dem Umlösen aus Pyridin bei 231°C unter Zersetzung schmelzen.

Beispiel 2:

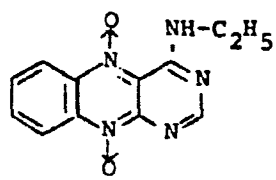

In entsprechender Weise, wie in Beispiel 1 angegeben, erhält man aus 2-Cyano-3-(dimethylamino-formylimino)-chinoxalin-di-N-oxid und Äthylamin in Dimethylformamid als Lösungsmittel bei 50°C in einer Ausbeute von 77 % der Theorie das 4-Äthylamino-Pyrimido-(4,5-b)-chinoxalin-5,10-dioxid von Fp. 170°C.

Patentansprüche

1. Bakterizide Mittel für den Pflanzenschutz, gekennzeichnet durch einen Gehalt an mindestens einem 4-Alkylamino-pyrimido-(4,5-b)-chinoxalin-5,10-dioxid der allgemeinen Formel

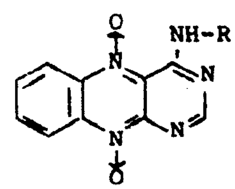

(I)

in welcher

R für Methyl oder Äthyl steht.

2. Verfahren zur Bekämpfung von pflanzenschädigenden Bakterien, dadurch gekennzeichnet, daß man 4-Alkylamino-pyrimido-(4,5-b)-chinoxalin-5,10-dioxide gemäß Anspruch 1 auf Bakterien oder ihren Lebensraum einwirken läßt.

3. Verwendung von 4-Alkylamino-pyrimido-(4,5-b)-chinoxalin-5,10-dioxiden gemäß Anspruch 1 zur Bekämpfung von pflanzenschädigenden Bakterien.

4. Verfahren zur Herstellung von bakteriziden Mitteln für den Pflanzenschutz, dadurch gekennzeichnet, daß man 4-Alkylamino-pyrimido-(4,5-b)-chinoxalin-5,10-dioxide gemäß Anspruch 1 mit Streckmitteln und/oder oberflächenaktiven Mitteln vermischt.

0001423

Nummer der Anmeldung

EP 78 10 097

Europäisches Patentamt

## EUROPÄISCHER RECHERCHENBERICHT

### EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| | US - A - 3 985 879 (F. SENG et al.)<br>* Spalte 1, Zeilen 11-15; Beispiele 1 und 2 * | 1-4 |
| D | & DE - A - 2 034 476 | |
| | ---- | |

**KLASSIFIKATION DER ANMELDUNG (Int.Cl.²)**

A 01 N 9/22<br>C 07 D 487/04//<br>(C07 D 487/04<br>241/00<br>239/00)

**RECHERCHIERTE SACHGEBIETE (Int. Cl.²)**

A 01 N 9/22

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument

&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 15-12-1978 | DECORTE |